Europäisches Patentamt

European Patent Office

Office européen des brevets

(11) Veröffentlichungsnummer: **0 217 975 B1**

# (12) EUROPÄISCHE PATENTSCHRIFT

(45) Veröffentlichungstag der Patentschrift: 04.12.91

(51) Int. Cl.⁵: **A61K 33/00, A61K 7/48**

(21) Anmeldenummer: **85112766.2**

(22) Anmeldetag: **08.10.85**

Die Akte enthält technische Angaben, die nach dem Eingang der Anmeldung eingereicht wurden und die nicht in dieser Patentschrift enthalten sind.

(54) **Salzmischung zur Behandlung der Schuppenflechte (Psoriasis) und anderer Hautkrankheiten.**

(43) Veröffentlichungstag der Anmeldung:
15.04.87 Patentblatt 87/16

(45) Bekanntmachung des Hinweises auf die Patenterteilung:
04.12.91 Patentblatt 91/49

(84) Benannte Vertragsstaaten:
**AT BE CH DE FR GB IT LI NL SE**

(56) Entgegenhaltungen:
WO-A-84/04885
DE-A- 2 124 735

**CHEMICAL ABSTRACTS, Band 103, Nr. 15, 14. Oktober 1985, Seite 82, Nr. 116251g, Columbus, Ohio, US; J. SHANI et al.: "Skin penetration of minerals in psoriatics and guinea pigs bathing in hypertonic salt solutions", & PHARMACOL. RES. COMMUN. 1985, 17(6), 501-12**

**K.S. SPIEGLER: "Salt-water purification", 1962, John Wiley & Sons Inc., New York, US.**

(73) Patentinhaber: **PSORI-MED AG**
**Dufourstrasse 101**
**CH-8034 Zürich(CH)**

(72) Erfinder: **Biener, Hans Prof. Dr.**
**Heilmannstrasse 21**
**W-8000 München 71(DE)**

(74) Vertreter: **Liesegang, Roland, Dr.-Ing. et al**
**FORRESTER & BOEHMERT Widenmayerstrasse 4**
**W-8000 München 22(DE)**

**Beschreibung**

In Europa und den USA leiden zwischen 2 und 3 % der Bevölkerung an Psoriasis vulgaris. Da es sich dabei um einen genetischen Defekt handelt, kann keine therapeutische Methode eine Dauerheilung gewährleisten. Alle Heilverfahren zielen deshalb auf Symptomfreiheit für möglichst lange Zeit, d.h. in der Regel 6 - 12 Monate.

Die erfahrungsgemäß immer wiederkehrende Psoriasis, die in besonders schweren Fällen sogar lebensbedrohend sein kann, erfordert meist eine Behandlung der Patienten mit Unterbrechungen über die ganze Lebenszeit, wobei das Auftreten bereits in der Jugend oft zu Therapiezeiten über mehrere Jahrzehnte zwingt. Mit der Anwendungsdauer steigt naturgemäß das Behandlungsrisiko, da übliche Mittel wie die Corticosteroide kumulierende Langzeit-Nebeneffekte aufweisen. Entsprechendes gilt für Dermatosen und sogar Melanome - der sogenannte Lichthautkrebs - bei ausgedehnter, jahrelanger UV-Bestrahlung.

Deshalb hat sich in den letzten Jahren die Suche nach natürlichen Heilverfahren ohne unkalkulierbare Risiken durch Nebeneffekte verstärkt. Das verbreitetste Beispiel ist die Badebehandlung am Toten Meer, die schon im Altertum angewandt wurde.

Sie hat drei wesentliche Nachteile: Erstens machen die hohen Kosten Reise und Aufenthalt nur für wenige erschwinglich. Zweitens belasten das heiße, ungewohnte Klima und die Strapazen der Reise besonders ältere Patienten und können der Therapie entgegenwirken. Drittens werden bei der Totes Meer-Behandlung immer wieder Allergien oder Reaktionen beobachtet, die auf den Gehalt an Verunreinigungen, organischen Verbindungen etc. zurückzuführen sind. Bekanntlich hat das Tote Meer keinen Abfluß und nimmt seit Jahrtausenden Abfälle, in neuerer Zeit auch Hotel- und Industrieabwässer und schließlich die Hautabschilferungen u.s.w. von Tausenden kranker Badenden irreversibel auf. Seit 1978 gilt das Tote Meer als chemisch "umgekippt". Ein biologischer Abbau findet, wegen des Fehlens geeigneter Mikroorganismen, in der Hochkonzentrierten Salzlösung nicht statt.

Ein Transport des Toten Meer-Wassers kommt aus Kostengründen praktisch nicht in Frage, auch nicht ein Eindampfen zur Trockne wegen des Energieverbrauchs und der Korrosionsprobleme. Auch wäre dadurch das Problem der Verschmutzung, Allergie- und sonstigen Reaktionen nicht zu lösen. Gleiches gilt von Salzen, die am Toten Meer aus Salzstöcken, Eindampfrückständen oder natürlichen Ablagerungen gewonnen werden. Sie unterscheiden sich von dem Anmeldungsgegenstand u. a. dadurch, daß es sich um natürliche Produkte mit wechselnder Zusammensetzung und vor allem wechselnder Reinheit handelt, die undefinierte Stoffe enthalten oder jederzeit enthalten können.

Bekanntlich muß immer ein bestimmter Prozentsatz von Patienten die Behandlung am Toten Meer einschränken oder gar abbrechen, da außer Allergien weitere Beschwerden wie z. B. Kreislaufprobleme auftreten oder die Krankheit sich sogar noch verschlimmert.

Aufgabe der vorliegenden Erfindung war es demnach, eine Lösung zu finden, die bei vergleichbarer Wirksamkeit alle diese Nachteile vermeidet.

Die Lösung der Aufgabe ergab sich in überraschender Weise durch die in den Ansprüchen dargelegten Mittel bzw. durch das dort beanspruchte Verfahren zu ihrer Herstellung.

Das Baden in Lösungen von Salzmischungen der erfindungsgemäßen Zusammensetzung bzw. ihr Aufbringen auf die Haut führte zu unerwartet bemerkenswerten Heileffekten. Besonders überraschend aber ist, daß selbst bei Hunderten bisher behandelter Patienten kein einziger Fall von Allergie und dergleichen auftrat und aus den beim Toten Meer geschilderten Gründen noch keine einzige Behandlung abgebrochen werden mußte. Dieser so günstige Befund war keinesfalls zu erwarten und stellt eine erhebliche Verbesserung der Heilmethode dar.

Die erfindungsgemäße Salzmischung wurde klinisch getestet und ist seit dem 26. Oktober 1984 vom deutschen Bundesgesundheitsamt als apothekenpflichtiges Arzneimittel zugelassen.

Entsprechend positive Auswirkungen hat die erfindungsgemäße Salzmischung bei Akne, die bei rd. 15 % der europäischen Jugendlichen im Alter von 12 - 25 Jahren so stark auftritt, daß eine Therapie angebracht ist, sowie bei z. B. Neurodermitis, Ichthyosis und anderen Hautkrankheiten.

Zur Unterstützung der Heilbehandlung, die balneologisch möglicherweise auch auf eine Erhöhung der Serum-Bromgehalte zurückgeht, kann bei der Anwendung des erfindungsgemäßen Mittels auch eine Bestrahlung mit UV-Licht, Schall oder elektromagnetischer Strahlung beliebiger Wellenlänge herangezogen werden. Auch können vorteilhaft zur Unterstützung der Therapie bekannte Wirkstoffe für die Hautbehandlung eingesetzt werden wie beispielsweise Teer und -derivate, Salicylsäure und -derivate oder auch rückfettende und hautschützende Präparate, Antiseptika, Antimykotica, Dermatica und/oder lichtabsorbierende Substanzen. Diese Zusätze können auch direkt in die Salzmischung eingearbeitet werden.

Desgleichen ist es je nach Hauttyp und -beschaffenheit der Patienten sowie Badtemperatur und -dauer vorteilhaft, die Konzentration der erfindungsgemäßen Salzlösungen zu variieren. Beispielsweise wird man

2

EP 0 217 975 B1

Patienten mit starken Hautläsionen nur eine Anfangs-Salzkonzentration von 0,5 - 8 Gewichts-% zumuten, während es in anderen Fällen angebracht sein kann, die Wirkung durch Konzentrationen bis etwa 34 Gewichts-% zu beschleunigen.

Zur leichteren Dosierung kann die erfindungsgemäße Salzmischung auch in Granulat- oder Tablettenform (ggf. mit Sprengmitteln) angewendet werden oder auch in konzentrierter Lösung, die vor Gebrauch zu verdünnen ist.

Eine besonders vorteilhafte Anwendungsform besteht darin, daß anstelle der flüssigen Salzlösung diese in Gelform appliziert wird. Dadurch wird weit weniger Sustanz als beim Baden benötigt, und man kann gezielt nur bestimmte Körperstellen behandeln, ohne daß andere mit dem Mittel in Berührung kommen. Dasselbe gilt für die Anwendung von Kompressen.

Zur Herstellung des Salzgels wird die Salzlösung durch Zusätze von z. B. organischen Polymeren geliert, wie sie in den deutschen Offenlegungsschriften DE 31 03 499 A 1 oder 34 32 573 A 1 beschrieben sind. Eine andere, besonders bevorzugte Möglichkeit ist die Verwendung von Celluloseestern oder -ethern, von denen bereits 1 - 2 Gewichts-% zur Gelierung ausreichen. Das Gel kann dabei transparent bleiben, was bei zusätzlicher UV-Bestrahlung Vorteile bietet, oder auch eingefärbt werden, z. B. schwarz zur Absorption von IR-Strahlung. Aus dem gleichen Grunde kann auch die Salzmischung eingefärbt werden.

Als sehr vorteilhaft für die Stabilität und Hautverträglichkeit hat sich ein Zusatz von 1 - 20 % Glykolen und insbesondere von Glycerin erwiesen.

Außer in Gelform kann die erfindungsgemäße Salzmischung auch dispergiert oder als Lösung emulgiert oder z. B. in handelsüblichen Salbengrundlagen eingesetzt werden.

**Patentansprüche**

1. Lösung einer Salzmischung zur Behandlung der Schuppenflechte (Psoriasis) und anderer Hautkrankenheiten wie Akne, Neurodermitis, Ichthyosis, dadurch **gekennzeichnet,** daß die lösung eine konzentration von mindestens 0,5 und höchstens 34 Gewichtsprozent aufweist und daß die zur Herstellung der Lösung benutzte Salzmischung folgende Zusammensetzung aufweist (in g/kg Salzmischung, Rest zu 1.000 g ist Kristallwasser):

| Magnesium | 20 -285 | Chlorid | 20 -730 |
| Natrium | 11 -266 | Bromid | 0,2 - 29 |
| Calcium | 2 -235 | Sulfat | 0,2 - 22 |
| Kalium | 2 - 95 | Borat | 0.05 - 14 |
| Strontium | 0,02 - 10,5 | Silikat | 0,02 - 14 |
| Eisen | 0,02 - 8,5 | Fluorid | 0,001 - 11 |
| Aluminium | 0,001 - 6,0 | Jodid | 0,001 - 9,5 |
| Zink | 0.001 - 2,5 | Carbonat | 0.0002- 9,0 |
| Lithium | 0,001 - 2,0 | Hydrogencarbonat | 0.0001 - 8,5 |

wobei eine lösungskonzentration bei 24,06% sowie folgende Zusammensetzung der Salzmischung vom Schutz ausgeschlossen sein sollen:

3

| | |
|---|---|
| Magnesiumchlorid 6 $H_2O$ | 581,04 |
| Natriumchlorid | 213,05 |
| Calciumchlorid 2 $H_2O$ | 108,46 |
| Magnesiumchlorid 2 $H_2O$ | 50,36 |
| Kaliumchlorid | 27,11 |
| Natriumbromid | 11.62 |
| Aluminiumsulfat 18 $H_2O$ | 0,2443 |
| Natriumtetraborat 10 $H_2O$ | 0,2269 |
| EisenIlsulfat 2 $H_2O$ | 0,1047 |
| Natriumfluorid | 0,0942 |
| Natriummetasilikat $H_2O$-frei | 0,0873 |
| Kaliumiodid | 0,0419 |
| Zinkchlorid $H_2O$-frei | 0,0175 |
| getrocknetes Magnesiumsulfat | 5,373 |
| Strontiumchlorid 6 $H_2O$ | 1,55 |
| Natriumhydrogencarbonat | 0,39 |
| Lithiumchlorid | 0,19 |
| Natriumcarbonat | 0,04 |

2. Mittel zur Behandlung der Psoriasis und anderer Hautkrankheiten nach Anspruch 1, dadurch **gekennzeichnet,** daß die Salzmischung folgenden Zusammensetzung aufweist

(in g/kg Salzmischung, Rest zu 1.000 g ist Kristallwasser):

| | | | |
|---|---|---|---|
| Magnesium | 55 - 108 | Chlorid | 340 - 550 |
| Natrium | 51 - 126 | Bromid | 1,5 - 15 |
| Calcium | 19 - 36 | Sulfat | 1,1 - 9 |
| Kalium | 10 - 21 | Borat | 0,4 - 3 |
| Strontium | 0,2 - 2 | Silikat | 0,1 - 2,9 |
| Eisen | 0,18 - 1,9 | Fluorid | 0,1 - 2,2 |
| Aluminium | 0,006 - 1,2 | Jodid | 0,1 - 2,0 |
| Zink | 0,02 - 0,8 | Carbonat | 0,01 - 1,0 |
| Lithium | 0,004 - 0,7 | Hydrogencarbonat | 0,01 - 1,0 |

3. Mittel zur Behandlung der Psoriasis und anderer Hautkrankheiten nach Anspruch 1 und 2, dadurch **gekennzeichnet,** daß die Salzmischung synthetisch, d.h. durch Vermischen von Salzen definierter Reinheit hergestellt wird.

4. Verfahren zur Herstellung der Salzmischung nach Anspruch 1 bis 3, dadurch **gekennzeichnet,** daß die Komponenten aus Salzen definierter Reinheit entsprechend der beanspruchten Zusammensetzung vermischt werden.

5. Mittel nach einem der Ansprüche 1 bis 3, dadurch **gekennzeichnet,** daß die Konzentration der Lösung zwischen 7 und 26 Gew. -% liegt.

6. Mittel nach einem der Ansprüche 1 bis 5, dadurch **gekennzeichnet,** daß die Lösung in Gel-Form vorliegt.

7. Mittel nach einem der Ansprüche 1 bis 3, 5 oder 6, dadurch **gekennzeichnet,** daß die Salzmischung oder das Gel weitere Wirkstoffe für die Hautbehandlung enthält.

Claims

1. A solution of a salt mixture for the treatment of psoriasis and other skin diseases such as acne,

EP 0 217 975 B1

neurodermatitis, ichthyosis, characterised in that the solution has a concentration of at least 0.5 and not more than 34% by weight and in that the salt mixture used for preparation of the solution has the following composition (in g/kg salt mixture, remainder up to 1000 g is water of crystallisation):

| Magnesium | 26 - 265 | Chloride | 26 - 756 |
|---|---|---|---|
| Sodium | 11 - 266 | Bromide | 0,2 - 29 |
| Calcium | 2 - 235 | Sulphate | 0,2 - 22 |
| Potassium | 2 - 95 | Borate | 0,05 - 14 |
| Strontium | 0,02 - 10,5 | Silicate | 0,02 - 14 |
| Iron | 0,02 - 0,5 | Fluoride | 0,001 - 11 |
| Aluminium | 0,001 - 6,0 | Iodide | 0,001 - 9,5 |
| Zinc | 0,001 - 2,5 | Carbonate | 0,0002 - 9,5 |
| Lithium | 0,001 - 2,0 | Hydrogen carbonate | 0,0001 - 8,5 |

a solution concentration of 24.06% and the following composition of the salt mixture being excluded from protection:

| | |
|---|---|
| Magnesium chloride 6 $H_2O$ | 581.04 |
| Sodium chloride | 213.05 |
| Calcium chloride 2 $H_2O$ | 108.46 |
| Magnesium chloride 2 $H_2O$ | 50.36 |
| Potassium chloride | 27.11 |
| Sodium bromide | 11.62 |
| Aluminium sulphate 18 $H_2O$ | 0.2443 |
| Sodium tetraborate 10 $H_2O$ | 0.2269 |
| Iron (II) sulphate 2 $H_2O$ | 0.1047 |
| Sodium fluoride | 0.0942 |
| Sodium metasilicate, anhydrous | 0.0873 |
| Potassium iodide | 0.0419 |
| Zinc chloride, anhydrous | 0.0175 |
| Dried magnesium sulphate | 5.373 |
| Strontium chloride 6 $H_2O$ | 1.55 |
| Sodium hydrogen carbonate | 0.39 |
| Lithium chloride | 0.19 |
| Sodium carbonate | 0.04 |

2. An agent for the treatment of psoriasis and other skin diseases according to claim 1, characterised in that the salt mixture has the following composition (in g/kg salt mixture, remainder up to 1000 g is water of crystallisation) :

| Magnesium | 55 - 108 | Chloride | 340 - 550 |
|---|---|---|---|
| Sodium | 51 - 126 | Bromide | 1.5 - 15 |
| Calcium | 19 - 36 | Sulphate | 1.1 - 9 |
| Potassium | 10 - 21 | Borate | 0.4 - 3 |
| Strontium | 0.2 - 2 | Silicate | 0.1 - 2.9 |
| Iron | 0.10 - 1.9 | Fluoride | 0.1 - 2.2 |
| Aluminium | 0.006 - 1.2 | Iodide | 0.1 - 2.0 |
| Zinc | 0.02 - 0.8 | Carbonate | 0.01 - 1.0 |
| Lithium | 0.004 - 0.7 | Hydrogen carbonate | 0.01 - 1.0 |

3. An agent for the treatment of psoriasis and other skin diseases according to claims 1 and 2, characterised in that the salt mixture is prepared by synthesis, i.e. by mixing salts of defined purity.

5

4. A process for the preparation of the salt mixture according to claims 1 to 3, characterised in that the components are blended from salts of defined purity in accordance with the composition claimed.

5. An agent according to any one of claims 1 to 3, characterised in that the concentration of the solution is between 7 and 26% by weight.

6. An agent according to any one of claims 1 to 5, characterised in that the solution is presented in gel form.

7. An agent according to any one of claims 1 to 3, 5 or 6, characterised in that the salt mixture or the gel contains other active substances for skin treatment.

**Revendications**

1. Solution d'un mélange de sels pour le traitement du psoriasis et d'autres maladies de la peau telles que l'acné, les neurodermites, l'ichthyosis, caractérisée en ce que la solution est à une concentration d'au moins 0,5 et au maximum de 34 % en poids et en ce que le mélange de sels utilisé pour la préparation de la solution est à la composition suivante (en g/kg du mélange de sels, le solde à 1000 g consistant en eau de cristallisation) :

| magnésium | 20 à 285 | chlorure | 20 à 750 |
|---|---|---|---|
| sodium | 11 à 266 | bromure | 0,2 à 29 |
| calcium | 2 à 235 | sulfate | 0,2 à 22 |
| potassium | 2 à 95 | borate | 0,05 à 14 |
| strontium | 0,02 à 10,5 | silicate | 0,02 à 14 |
| fer | 0,02 à 8,5 | fluorure | 0,001 à 11 |
| aluminium | 0,001 à 6,0 | iodure | 0,001 à 9,5 |
| zinc | 0,001 à 2,5 | carbonate | 0,0002 à 9,0 |
| lithium | 0,001 à 2,0 | bicarbonate | 0,0001 à 9,5 |

la concentration de 24,06 % pour la solution et la composition ci-après du mélange de sels étant exclues de la protection :

| | |
|---|---|
| chlorure de magnésium, 6 $H_2O$ | 581,04 |
| chlorure de sodium | 213,05 |
| chlorure de calcium, 2 $H_2O$ | 108,46 |
| chlorure de magnésium, 2 $H_2O$ | 50,36 |
| chlorure de potassium | 27,11 |
| bromure de sodium | 11,62 |
| sulfate d'aluminium, 18 $H_2O$ | 0,2443 |
| tétraborate de sodium, 10 $H_2O$ | 0,2269 |
| sulfate de fer-II, 2 $H_2O$ | 0,1047 |
| fluorure de sodium | 0,0942 |
| métasilicate de sodium anhydre | 0,0873 |
| iodure de potassium | 0,0419 |
| chlorure de Zinc anhydre | 0,0175 |
| sulfate de magnésium sec | 5,373 |
| chlorure de strontium, 6 $H_2O$ | 1,55 |
| bicarbonate de sodium | 0,39 |
| chlorure de lithium | 0,19 |
| carbonate de sodium | 0,04 |

2. Produit pour le traitement du psoriasis et d'autres maladies de la peau selon la revendication 1, caractérisé en ce que le mélange de sels est à la composition suivante (en g/kg du mélange de sels, le solde à 1000 g consistant en eau de cristallisation) :

| magnésium | 55 à 108 | chlorure | 340 à 550 |
| sodium | 51 à 126 | bromure | 1,5 à 15 |
| calcium | 19 à 36 | sulfate | 1,1 à 9 |
| potassium | 10 à 21 | borate | 0,4 à 3 |
| strontium | 0,2 à 2 | silicate | 0,1 à 2,9 |
| fer | 0,18 à 1,9 | fluorure | 0,1 à 2,2 |
| aluminium | 0,006 à 1,2 | iodure | 0,1 à 2,0 |
| Zinc | 0,02 à 0,8 | carbonate | 0,01 à 1,0 |
| lithium | 0,004 à 0,7 | bicarbonate | 0,01 à 1,0 |

3. Produit pour le traitement du psoriasis et d'autres maladies de la peau selon les revendications 1 et 2, caractérisé en ce que le mélange de sels a été préparé par synthèse, c'est-à-dire par mélange de sels à pureté définie.

4. Procédé de préparation du mélange de sels selon les revendications 1 à 3, caractérisé en ce que l'on mélange les composants consistant en sels à pureté définie conformément à la composition revendiquée.

5. Produit selon l'une des revendications 1 à 3, caractérisé en ce que la concentration de la solution se situe entre 7 et 26 % en poids.

6. Produit selon l'une des revendications 1 à 5, caractérisé en ce que la solution est à l'état de gel.

7. Produit selon l'une des revendications 1 à 3, 5 ou 6, caractérisé en ce que le mélange de sels ou le gel contient d'autres substances actives pour le traitement de la peau.